# EUROPEAN PATENT APPLICATION

(11) **EP 4 286 397 A1**
(43) Date of publication of application: **06.12.2023**
(21) Application number: 22176765.0
(22) Date of filing: 01.06.2022

(54) **HYDROGEL-FORMING PROTEINS**

(71) Applicant: ETH Zurich, 8092 Zurich (CH); Zhaw - Zürcher Hochschule für angewandte Wissenschaften, 8820 Wädenswil (CH)
(72) Inventor: HILVERT, Donald, 8006 Zürich (CH); EDWARDSON, Thomas, 8048 Zürich (CH); TIBBITT, Mark, 8004 Zürich (CH); GUZZI, Elia, 8902 Urdorf (CH); HORI, Mao, 8047 Zürich (CH); OTA, Yusuke, Tokyo, 190-0013 (JP); DRANSEIKIENE, Dalia, 8001 Zürich (CH); PETERS, Christin, 8804 Au (CH); RAGUZ NAKIC, Zrinka, 8050 Zürich (CH); MATTLI, Kevin, 7310 Bad Ragaz (CH)

(57) **Abstract**

The present invention relates to a hydrogel-forming protein comprising at least three self-assembling aggregation domains and at least two hydrophilic non-self-assembling linker domains, wherein the self-assembling aggregation domains are covalently interconnected by the hydrophilic non-self-assembling linker domain, either directly or via a peptide linker, as well as a bioink comprising of the hydrogel-forming peptide of any one of the preceding claims for three-dimensional (3D) bio-fabrication.

## Description

The present invention relates to the field of bioinks, in particular to a novel class of hydrogel-forming proteins comprising self-assembling aggregation domains, and hydrophilic non-self-assembling linker domain, and to the use thereof as bioinks for biofabrication.

### RELATED ART

Biofabrication is characterized by the exploitation of automated procedures to directly create a 3D arrangement of cells, often with the aid of biomaterials (J. Groll et al, Biofabrication 11, 2019, 013001). But the lack of variability of bioinks suitable for 3D bioprinting has been identified as one drawback, limiting rapid progress in the field (J. Groll et al, Biofabrication 11, 2019, 010201, and papers cited therein).

A key challenge of 3D bioprinting is the development of functional bioink material. In many cases, natural proteins need to be chemically modified and cross-linked in order to achieve mechanical properties sufficient for bioprinting. WO 2018/225076 A1 describes the use of a recombinant human Collagen type I for additive manufacturing which is produced in a transgenic tobacco plant. Others bioinks, such as peptide-based bioinks, are prepared via chemical synthesis (S. Boothroyd, A. Saiani, A. F. Miller, Biopolymers 2013, 101, 6, 669-680). Many of these products have insufficient rheological, mechanical and biological properties, which limits their utility as bioinks.

Hydrogels are mentioned in the art as a natural choice of bioink materials for cellular printing, because they can provide a highly hydrated and permeable 3D polymeric structure conductive to cellular anchorage and metabolic activities. However, owing to the stringent cellular bioprinting requirements regarding parameters, such as temperature, pH, pressure, physical forces, and osmolarity for safe-guarding the vitality of printed cells and bioactivity of the printed structure, a widely accepted optimal bioink hydrogel for cellular bio-printing has not yet been developed (L. Bian, APL Bioeng. 4, 030401, 2020).

WO 2005/014615 A1 discloses that it is possible to modify self-assembling peptides by incorporating an additional domain that does not self-assemble, while still permitting assembly of the self-assembling portion. However, these modifications resulted in a very significant drop of the mechanical properties of the gels.

Olsen et al. (Macromolecules 2010, 43, 21, 9094-9099) describe telechelic proteins P-Cₓ-P constructed from two helical endblocks (denoted P) linked by 10 or 30-repeats of the flexible nonapeptide sequence C. The coiled-coil P domains associate into pentameric bundles, forming physical crosslinks in the gels. Despite relatively high protein concentrations in Olsen et al., the storage modulus of the hydrogel obtained from protein PC₃₀P was modest.

Thus, there is still a high need for improved peptide- or protein-based bioinks.

### SUMMARY OF THE INVENTION

The present invention relates in a first aspect to a novel hydrogel-forming protein comprising at least three self-assembling aggregation domains (A domains), at least two hydrophilic non-self-assembling linker domain (B domains), wherein the self-assembling aggregation domains are covalently interconnected by the hydrophilic non-self-assembling linker domain, either directly or via a peptide linker.

In a further aspect, the invention relates to a hydrogel-forming protein comprising at least two self-assembling aggregation domains (A domains) and at least one hydrophilic non-self-assembling linker domain (B domain), wherein the self-assembling aggregation domains are covalently interconnected by the hydrophilic non-self-assembling linker domain, either directly or via a peptide linker.

In a preferred embodiment, at least one, more preferably all, of the self-assembling aggregation domains are amphiphilic beta-sheet forming peptides.

The present invention relates in a further aspect to the use of the hydrogel-forming protein of the invention as bioink, preferably for three-dimensional (3D) biofabrication or in a method of 3D biofabrication. The present invention relates in a further aspect to a method for 3D biofabrication using the hydrogel-forming peptide of the present invention. In another aspect, the invention refers to a bioink comprising the hydrogel-forming peptide of the invention.

In another aspect, the invention refers to a method for preparing the hydrogel-forming peptide of the present invention.

In a further aspect, the invention relates to a protein comprising a hydrophilic non-self-assembling linker domain (domain B) comprising at least one linker repeat unit comprising, preferably consisting of, the sequence GXGSGSG (SEQ ID NO: 70) or GXGSGSGRGDS (SEQ ID NO: 71), in which X represents a charged amino acid such as lysine, arginine, aspartic acid or glutamic acid; or a polar amino acid, such as serine, homoserine, histidine, threonine, glutamine, or asparagine

Protein-based hydrogels are a useful platform for tissue engineering as they resemble ECM constituents. Recombinant protein expression allows control of molecular structure and scalability of production compared to purification of proteins from their native sources. The protein-based hydrogels of the invention are modular de novo protein system with separate modules for either bioactivity or self-assembly. A self-assembling domain is based on amphiphilic peptide sequence forming beta-sheet structures. The bioactive hydrophilic non-self-assembling linker domains of the protein has cell adhesion motives embedded within a flexible and water-soluble peptide sequence. The resulting supramolecular protein hydrogel possesses suitable properties for processing via injection based techniques such as biofabrication.

The hydrogel-forming proteins of the invention showed suitable mechanical and rheological properties (i.e., plateau modulus of 1-25 kPa, a high degree of shear-thinning and self-healing, and apparent yield stress) for extrusion-based 3D printing and thus enables flow and shape retention and printing in self-supporting multi-layered structures. In addition, the hydrogel-forming proteins of the invention present cell-binding domains for cell adhesion, allow for biodegradation and resorption, and do not exhibit cytotoxicity. Cell spreading was observed, preferably in low concentration gels, made from the hydrogel-forming proteins of the invention. Thus, recombinant engineered proteins of the invention can be used as an effective strategy to formulate hydrogels and bioinks, suitable for biofabrication.

### DESCRIPTION OF FIGURES

FIGURE 1: SDS-PAGE analysis of OD-normalized samples from E. coli BL21(DE3) expressing PB1. Different conditions have been tested, soluble (S) and insoluble (I) phase were applied on the gel. R/2-Glc = R/2 minimal medium with glucose as carbon source. R/2-Gly = R/2 minimal medium with glycerol as carbon source. TB = Terrific broth. Autolnd = Autoinduction medium.
FIGURE 2A: ATR-FTIR spectra of PB1 samples prepared from lyophilised non-gelled protein solution and gelled hydrogel indicated a higher degree of beta-sheet structures in the hydrogel case, suggesting that gel formation proceeds, at least in part, via beta-sheet formation.
FIGURE 2B: Top: TEM images of nanofibrillar PB0 (left) and PB1 (right) proteins after self-assembly at 3.6 mg mL⁻¹ concentration. Bottom: SEM images of 120 mg mL⁻¹ PB0 (left) and 106 mg mL⁻¹ PB1 (right) hydrogel nanostructure.
FIGURE 3: Plateau storage modulus (G'), obtained from oscillatory frequency sweeps (γ = 0.3 %, ω = 100-0.1 rad s⁻¹) for each protein sample, dependence on engineered protein concentration. For PB1 and PB2-H-tr, modulus was found to increase with concentration.

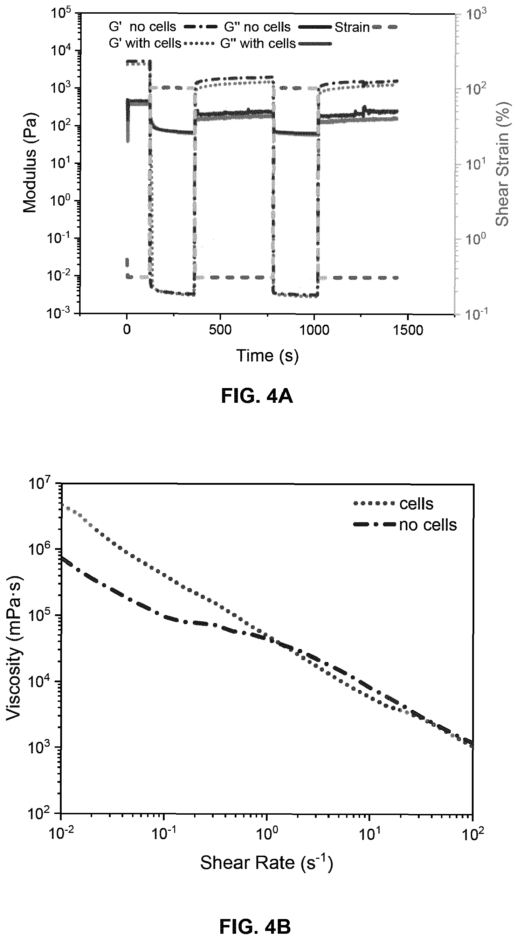

FIGURE 4A, 4B: Rheological properties of PB1 gel (5 wt%) samples before and after addition of NIH/3T3 cells (2 × 10⁶ cells mL⁻¹). Step strain measurements (stepping between strains of γ = 0.3 % (within LVR) and γ = 100 % (beyond LVR, breaking of the material), ω = 10 rad s⁻¹) were used to record changes in storage (G') and loss (G") moduli upon changing strain and evaluate material self-healing (Figure 4A). Overall, the addition of cells in the PB1 protein bioink slightly decreased the shear storage modulus (G'_{cells} ≈ 3.9 kPa, vs. G'_{no cells}≈ 4.9 kPa; ΔG' = 1 kPa), however, it did not impact (Figure 4A) self-healing (evaluated by step strain measurements at γ = 0.3 % and γ = 100 %) or (Figure 4B) the shear-thinning (evaluated by rotational shear rate δγ/δt = 0.01-100 s⁻¹ measurements).
FIGURE 5: PB1 gel (47 mg mL⁻¹; pH 5.5-6) printed via hand extrusion through 30G nozzle. Scale bars, 5 mm
FIGURE 6: PB1 5 % gel printed samples, grid (left) and cylinder (middle and right).
FIGURE 7: Calcein AM live cell staining of NIH/3T3 cells seeded on 2D well-plate and PB1 gel surfaces to compare cell spreading after 24 hours in culture.
FIGURE 8: Cell cytoskeleton staining (stained green/light colored): actin labelling with phalloidin-iFluor488) images to investigate NIH/3T3 cell spreading in PB1 and PB2-PHSRN hydrogels after 7 days in culture.
FIGURE 9: Day 5 cytoskeleton staining (stained green/light colored): actin labelling with phalloidin Alexa Fluor 488) images to investigate hMSC spreading in PB2-H-tr hydrogels of different concentrations.
FIGURE 10A: Printed 3D samples with bioinks composed of 1 × 10⁶ cells mL⁻¹ hMSCs and (left) 87.5 mg mL⁻¹ initial concentration PB1 protein hydrogel and (right) 101 mg mL⁻¹ initial concentration PB2-H-tr protein.
FIGURE 10B: Live/Dead staining (Calcein AM/Ethidium Homodimer) of the hMSCs in 3D printed PB2-H-tr protein bioink samples after 14 and 22 days in culture.

### DETAILED DESCRIPTION OF THE INVENTION

Unless defined otherwise, all technical and scientific terms used herein have the same meanings as commonly understood by one of ordinary skill in the art to which this invention belongs.

Throughout this specification and the claims which follow, unless the context requires otherwise, the word "comprise" or the word "include", and variations such as "comprises/includes" and "comprising/including", are to be understood to imply the inclusion of an element, stated integer, step or a group thereof but not the exclusion of any other element, stated integer, step or a group thereof.

As used in this specification and the appended claims, the singular forms "a", "an", and "the" include plural referents, unless the content clearly dictates otherwise.

The term "about" or "approximately" when used in connection with a numerical value is meant to encompass numerical values within a range having a lower limit that is 0-10 % smaller than the indicated numerical value and having an upper limit that is 0-10 % larger than the indicated numerical value. The term "about" or "approximately" means preferably ±10 %, more preferably ±5 %, again more preferably ±3 % or most preferably ±0 % (referring to the given numeric value, respectively). In each of the invention embodiments, "about" can be deleted. All ranges of values disclosed herein, should refer and include to any and all values falling within said range including the values defining the range. The terms "reduce", "inhibit" or "decrease" or the like, as used herein, include a just detectable reduction but also a reduction down to zero (reduction by 100 %).

A peptide or peptide moiety, as defined herein, is any peptide-bond-linked chain of amino acids, regardless of length, secondary and tertiary structure, number of subunits or posttranslational modification. Thus, the term "peptide" of "peptide moiety" is to be understood as covering the terms "polypeptide", "protein", "amino acid chain" and "polypeptide chain". Amino acids included in the peptide of the invention are proteinogenic, non-proteinogenic and synthetic amino acids. Peptides can be an open linear peptide chain or cyclic peptides; and may include at least one chemical modification, such as lipidation, glycosylation and phosphorylation. Peptides can be produced by chemical synthesis, RNA translation and/or recombinant processes.

The term "amino acid", as used herein, refers to organic compounds containing the functional groups amine (-NH₂) and carboxylic acid (-COOH) and its zwitterions, typically and preferably, along with a side chain specific to each amino acid. The term "amino acid" typically and preferably includes amino acids that occur naturally, such as proteinogenic amino acids (produced by RNA-translation), non-proteinogenic amino acids (produced by other metabolic mechanisms, e.g. posttranslational modification), standard or canonical amino acids (that are directly encoded by the codons of the genetic code) and non-standard or non-canonical amino acids (not directly encoded by the genetic code). Naturally occurring amino acids include non-eukaryotic and eukaryotic amino acids. The term "amino acid", as used herein, also includes unnatural amino acids that are chemically synthesized; alpha- (α-), beta- (β-), gamma- (γ-) and delta- (δ-) etc. amino acids as well as mixtures thereof in any ratio; and, if applicable, any isomeric form of an amino acid, i.e. its D-stereoisomers (labelled with a lower-case initial letter) and L-stereoisomers (labelled with a capital initial letter) (alternatively addressed by the (R) and (S) nomenclature) as well as mixtures thereof in any ratio, preferably in a racemic ratio of 1:1. Amino acids in this invention are preferably in L-configuration, unless mentioned specifically as D-configuration. Amino acid can include one or more modifications and/or attached groups, for example protecting groups used for peptide synthesis.

The hydrogel-forming proteins of the invention are block co-polymers, which comprise at least three self-assembling aggregation domains (called herein also A domains) and at least two non-self-assembling linker domains (called herein also B domain).

In a first aspect, the invention relates to a hydrogel-forming protein comprising at least three self-assembling aggregation domains and at least two hydrophilic non-self-assembling linker domain, wherein the self-assembling aggregation domains are covalently connected by the hydrophilic non-self-assembling linker domains, either directly or via a peptide linker.

According to a preferred embodiment, the invention relates to a hydrogel-forming protein consisting of at least three self-assembling aggregation domains and at least two hydrophilic non-self-assembling linker domains, wherein the self-assembling aggregation domains are covalently connected by the hydrophilic non-self-assembling linker domains, either directly or via a peptide linker.

According to a preferred embodiment, the invention relates to a hydrogel-forming protein comprising three self-assembling aggregation domains and two hydrophilic non-self-assembling linker domains, wherein the self-assembling aggregation domains are covalently connected by the hydrophilic non-self-assembling linker domains, either directly or via a peptide linker. According to a preferred embodiment, the invention relates to a hydrogel-forming protein consisting of three self-assembling aggregation domains and two hydrophilic non-self-assembling linker domain, wherein the self-assembling aggregation domains are covalently connected by the hydrophilic non-self-assembling linker domains, either directly or via a peptide linker.

According to a very preferred embodiment, the hydrogel-forming peptide, comprising three self-assembling aggregation domains and two hydrophilic non-self-assembling linker domain, has a formula of A-B-A-B-A, wherein A is the self-assembling aggregation domain, covalently linked to B, either directly or via a peptide linker, and B is the hydrophilic non-self-assembling linker domain.

According to a very preferred embodiment, the hydrogel-forming protein comprises exactly three A domains and exactly two B domain, wherein each two of the A domains are covalently interconnected by one B domain, either directly or via a peptide linker. Preferably, said hydrogel-forming protein has a formula of A-B-A-B-A.

In a preferred embodiment, at least one, more preferably all, of said A domains are amphiphilic beta-sheet forming peptide domains.

In a further aspect, the invention relates to a hydrogel-forming protein comprising at least two self-assembling aggregation domains (A domains) and at least one hydrophilic non-self-assembling linker domain (B domain), wherein the self-assembling aggregation domains are covalently interconnected by the hydrophilic non-self-assembling linker domain, either directly or via a peptide linker. In a preferred embodiment, at least one, more preferably all, of the self-assembling aggregation domains are amphiphilic beta-sheet forming peptides.

According to a preferred embodiment, the invention relates to a hydrogel-forming protein consisting of at least two self-assembling aggregation domains and at least one hydrophilic non-self-assembling linker domains, wherein the self-assembling aggregation domains are covalently connected by the hydrophilic non-self-assembling linker domains, either directly or via a peptide linker.

According to a preferred embodiment, the invention relates to a hydrogel-forming protein comprising two self-assembling aggregation domains and one hydrophilic non-self-assembling linker domains, wherein the self-assembling aggregation domains are covalently connected by the hydrophilic non-self-assembling linker domains, either directly or via a peptide linker. According to a preferred embodiment, the invention relates to a hydrogel-forming protein consisting of two self-assembling aggregation domains and one hydrophilic non-self-assembling linker domain, wherein the self-assembling aggregation domains are covalently connected by the hydrophilic non-self-assembling linker domains, either directly or via a peptide linker.

According to a very preferred embodiment, the hydrogel-forming peptide comprising two self-assembling aggregation domains A and one hydrophilic non-self-assembling linker domain B, has a formula of A-B-A, wherein A is the self-assembling aggregation domain, covalently linked to B, either directly or via a peptide linker, and B is the hydrophilic non-self-assembling linker domain.

According to a very preferred embodiment, the hydrogel-forming protein comprises exactly two A domains and exactly one B domain, wherein the two A domains are covalently interconnected by the B domain, either directly or via a peptide linker. Preferably, said hydrogel-forming protein has a formula of A-B-A. In a preferred embodiment, at least one, more preferably all, of the self-assembling aggregation domains are amphiphilic beta-sheet forming peptides.

The following sections, especially the sections under items 1., 2. and 3., relate to all aspects of the invention described herein.

A and B domains alternate in the hydrogel-forming protein of the invention. Preferably, two of the self-assembling aggregation domains are covalently interconnected by one of the hydrophilic non-self-assembling linker domains, either directly or via one or more peptide linkers.

Preferably, two of the A domains are positioned at the N- and C-termini of the hydrogel-forming protein, wherein the N- and C terminal A domains are covalently interconnected each by the B domain, either directly or via a peptide linker.

Preferably, the covalent interconnections and connections in the hydrogel-forming proteins of the invention are peptide (amide) bonds.

The term "hydrogel" as used herein refers to a hydrophilic or amphiphilic polymer material that possesses a measurable volume fraction of water and that does not dissolve in aqueous solutions. Hydrogels are preferably prepared by chemical or physical crosslinking of polymer building blocks, such as peptide repeat units, homopolymers or copolymers, resulting a reticulated network structure that is prepared or swollen in an aqueous solution. Preferably, a hydrogel is capable of taking up large quantities of water, but is insoluble due to the presence of covalent chemical or physical (ionic, hydrophobic interactions, entanglements) crosslinks. The crosslinks provide a network structure and physical integrity. Hydrogels exhibit a thermodynamic compatibility with water that allow them to swell in aqueous media.

Herein, hydrogels are prepared via the self-assembly of engineering protein building blocks through beta-sheet forming polypeptide domains, leading to the formation of water-swollen polymer materials (hydrogels). The term "crosslinked" as used herein refers to a composition containing intramolecular and/or intermolecular crosslinks, whether arising through covalent or noncovalent bonding. "Noncovalent" bonding includes hydrogen bonding, Van der Waals forces, hydrophobic interactions, pi-interactions and electrostatic (ionic) bonding.

Preferably, a first self-assembling aggregation domain (A domain) is positioned at the N-terminus, a second A domain is positioned at the C-terminus of the hydrogel-forming protein, wherein the N- and/or C-terminal A domains are covalently connected to the hydrophilic non-self-assembling linker domains, either directly or via a peptide linker, and A and B domains alternate. Preferably, the N- and/or C-terminal A or B, preferably A domains, independently of each other, comprise at the N- and/or C terminus of the hydrogel-forming protein one or more functional oligomeric peptide stretches, such as a polyhistidine tag (His-tag; short amino acid stretch containing more than one consecutively linked histidines) or the hydrophilic peptide of sequence GTSGS (SEQ ID NO: 69) which aids solubility. In a preferred embodiment, the hydrogel-forming peptide of the invention comprises either at the N or C terminus a polyhistidine tag of SEQ ID NO: 77 (HHHHHHGSGS) and at the other terminus the hydrophilic peptide of SEQ ID NO: 69.

According to one embodiment, the hydrogel-forming peptides of the invention contain a protease cleavage site, preferably included in the hydrophilic linker domain. More preferably, said protease cleavage site is included between two linker repeat units of the hydrophilic linker domain. Alternatively, the protease cleavage site is included between the A and B domains.

According to one embodiment, the hydrogel-forming peptide of the invention comprises an N- or C-, preferably an N-terminal, affinity tag for chromatographic purification, such as a polyhistidine tag (His-tag). Preferably, said His-tag consists of 3-8, more preferably 6 histidine residues.

Preferably, the hydrogel-forming peptides of the invention have a length of 100 to 300 amino acids, preferably of 150 to 260 amino acids, more preferably 170 to 260 amino acids.

### 1. Self-assembling aggregation domains

According to a preferred embodiment, each of said self-assembling aggregation domains comprises, preferably consists of, at least two A repeat units that are preferably consecutively linked.

According to a preferred embodiment, at least one, preferably all, of the self-assembling aggregation domains (called herein also A domains or domains A) comprise, preferably consist of amphiphilic beta-sheet forming oligomeric peptide motifs, herein mentioned also as repeat units or A repeat units. The term "self-assembling" relates to establishment of physical crosslinks involving weak specific interactions (i.e., hydrogen bonds and π-π stacking) or strong non-specific interactions (i.e., electrostatic), α-helices, β-sheets, coiled-coils, or other naturally occurring motifs, preferably hydrogen bonds and electrostatic interactions.

The term "amphiphilic peptide" or "amphiphilic protein" as used herein refers to a peptide or protein that contains both hydrophobic and hydrophilic amino acids, which are often arranged in such a way to allow orthogonal interactions between hydrophobic residues and hydrophilic residues.

The self-assembling aggregation domains (A domains) preferably aggregate spontaneously into beta-sheets. The term "beta-sheets" or "beta-sheet forming peptides" as used herein refers to sequences of amino acids that have a propensity to form beta sheet structures. Beta sheets consist of beta strands connected laterally by preferably at least two or three intermolecular backbone hydrogen bonds between the amide functional groups, forming a preferably twisted, pleated sheet. A beta-strand is an oligomeric peptide stretch, called herein repeat unit A or repeat unit. Preferably its backbone is in an extended conformation.

The A domains consist preferably of alternating hydrophilic and hydrophobic amino acids that are capable of self-assembling to form an exceedingly stable beta-sheet macroscopic structure, e.g. in the presence of electrolytes, such as monovalent cations. The A domains are preferably complementary and structurally compatible, and their side-chains in the structure partition preferably into two faces, a polar face with charged ionic side chains and a nonpolar face with alanines or other hydrophobic groups. These ionic side chains are preferably self-complementary to one another in that the positively charged and negatively charged amino acid residues can form complementary ionic pairs.

Each of said A repeat units of the A domains is an oligomeric peptide having a certain motif and is repeated within the A domain. A repeat units of the A domain are preferably consecutively and covalently linked. In a preferred embodiment, said A repeat unit has a length of 8 or a multiple thereof, e.g. 16 or 24 amino acids. In another preferred embodiment, the A repeat units is preferably 3 to 10, most preferably 8 amino acids long.

Preferred amphiphilic beta-sheet forming peptides and A repeat units are described in Zhang S. Altman M., Reactive & Functional Polymers, 41, 91, 1999; WO 2005/014615, US 7,713,923 B2; US 8,901,084 B2; US 5,670,483; US 5,955,343; US 09/778,200; EP 1636250 B1, JP 5057781 B2; EP 3031466 B1; and L. M. De Leon Rodriguez, Y. Hemar, J. Cornish, M. A. Brimble, Chem. Soc. Rev. 2016, 45, 4797.

According to a preferred embodiment, in the hydrogel-forming protein of the invention, each of said A domains comprises, preferably consists of, at least two consecutively covalently linked A repeat units. Preferably, at least one, more preferably all of the A repeat units comprise amphiphilic beta-sheet forming peptide motifs. Preferably, at least one, more preferably all of the A repeat units consist of amphiphilic beta-sheet forming peptide motifs.

Preferred A repeat units are selected from the group consisting of AEAEAKAK (SEQ ID NO: 1), RADARADA (SEQ ID NO: 3), FEFEFKFK (SEQ ID NO: 11), KADAKADA (SEQ ID NO: 7) and of amphiphilic beta-sheet forming peptide sequences with other structural moduli mentioned in S. Zhang, M. Altman, Reactive & Functional Polymers, 41, 91, 1999, such as AEAKAEAK (SEQ ID NO: 2, modulus-I), RARADADA (SEQ ID NO: 4, modulus-II) or FEFKFEFK (SEQ ID NO: 10, modulus-I).

Other preferred examples of amphiphilic beta-sheet forming peptide sequences are listed in **Table 1** SEQ ID NO: 1-20).

**Table 1. Preferred amphiphilic beta-sheet forming peptide sequences**

| SEQ ID NO: | Name | Sequence | Modulus |
|---|---|---|---|
| 1 | EAKA8-II | AEAEAKAK | II |
| 2 | EAKA8-I | AEAKAEAK | I |
| 3 | RADA8-I | RADARADA | I |
| 4 | RADA8-II | RARADADA | II |
| 5 | RAEA8-I | RAEARAEA | I |
| 6 | RAEA16-I | RAEARAEARAEARAEA | I |
| 7 | KADA8-I | KADAKADA | I |
| 8 | KADA8-II | KAKADADA | I |
| 9 | EAH8-II | AEAEAHAH | II |
| 10 | EFK8-I | FEFKFEFK | I |
| 11 | EFK8-II | FEFEFKFK | II |
| 12 | ELK8-I | LELKLELK | I |
| 13 | ELK8-II | LELELKLK | II |
| 14 | EHK8-1 | HEHEHKHK | N/A |
| 15 | EVKV8-I | VEVKVEVK | I |
| 16 | KAE16-IV | KAKAKAKAEAEAEAEA | IV |
| 17 | EAK16-IV | EAEAEAEAKAKAKAKA | IV |
| 18 | RAD16-IV | RARARARADADADADA | IV |
| 19 | DAR16-IV | ADADADADARARARAR | IV |
| 20 | DAR16-I | DADADADARARARARA | IV |

If the ionic residues alternate with one positively and one negatively charged residue (-+-+-+-+), the peptide chains are described as "modulus I;" if the ionic residues alternate with two positively and two negatively charged residues (--++--++), the peptide chains are described as "modulus II." Both D- and L- amino acids may be used to produce peptide chains. They may be mixed in the same chain, or peptide compositions may be prepared having mixtures of individual chains that themselves only include D- and L- amino acids.

More preferably, at least one, preferably all, of the self-assembling A domains of the block-copolymer consist of oligomers of amphiphilic beta-sheet forming peptide sequence AEAEAKAK (SEQ ID NO: 1). Most preferably, at least one, preferably all of the self-assembling domains A of the block-copolymer consist of oligomers of amphiphilic beta-sheet forming peptide sequence AEAEAKAK.

According to a very preferred embodiment, the hydrogel-forming peptide comprises at least 3, more preferably 3 self-assembling aggregation domains. According to another preferred embodiment, the hydrogel-forming peptide comprises 2 self-assembling aggregation domains. Each A domain is covalently connected to a B domain.

According to a preferred embodiment, each of the aggregation domains has a length from about 16 to about 60. According to another preferred embodiment, each of the aggregation domains has a length from about 24 to about 50. Most preferably, each of the aggregation domains has a length from more preferably from about 24 to about 40. In a very specific preferred embodiment, each of the aggregation domains has a length from of about 40 amino acids.

According to a preferred embodiment, the self-assembling aggregation domains of one hydrogel-forming peptide differ from each other in the respective number of amino acids. In another more preferred embodiment, the self-assembling aggregation domains of one hydrogel-forming peptide differ from each other in the respective number of amino acids. According to a preferred embodiment, the self-assembling aggregation domains of one hydrogel-forming peptide are different from each other in the respective amino acid sequence. In another more preferred embodiment, the domains of one hydrogel-forming peptide are all structurally identical.

According to a preferred embodiment, each of said self-assembling aggregation domains comprises 2 to 8 A repeat units, more preferably 3 to 6 A repeat units, again more preferably 3 to 5 A repeat units, most preferably 3 or 5 A repeat units. Preferably, said A repeat units are consecutively linked. More preferably, said A repeat units are directly covalently and consecutively linked. According to another preferred embodiment, each of said self-assembling aggregation domains consists of 2 to 8 A repeat units, more preferably 3 to 6 A repeat units, again more preferably 3 to 5 repeat units, most preferably 3 or 5 A repeat units. Preferably, said A repeat units are consecutively linked. More preferably, said A repeat units are directly covalently and consecutively linked. According to a specific preferred embodiment, each of said self-assembling aggregation domains comprises, preferably consists of 5 A repeat units repeat units that are consecutively linked. Preferably, said A repeat units are directly covalently and consecutively linked.

According to a preferred embodiment, the repeat unit consists of amphiphilic beta-sheet forming peptide sequences. Preferably, the repeat unit has an amino acid sequence selected of the group consisting of SEQ ID NO: 1-20, more preferably of SEQ ID NO: 1-4, 7, 10 and 11. Most preferably, the repeat unit has an amino acid sequence of SEQ ID NO: 1.

According to a preferred embodiment, each of said self-assembling aggregation domains consists of 2 to 8, preferably 3 to 6, more preferably 3 to 5, again more preferably 3 or 5 consecutively linked repeat units, wherein said repeat units are selected of the group consisting of SEQ ID NO: 1-20. According to another preferred embodiment, each of said self-assembling aggregation domains consists of 2 to 8, preferably 3 to 6, more preferably 3 to 5, again more preferably 3 or 5 consecutively linked repeat units, wherein said repeat units are selected of the group consisting of SEQ ID NO: 1-4, 7, 10 and 11. According to another preferred embodiment, said self-assembling aggregation domain consists of (AEAEAKAK)ₙ, wherein n is an integer from 2 to 8, preferably from 3 to 6, more preferably from 3 to 5, most preferably 3 or 5. According to another preferred embodiment, each of said self-assembling aggregation domains comprises, preferably consists of 3 to 5 consecutively linked repeat units, wherein said repeat units are selected of the group consisting of SEQ ID NO: 1-20. According to another preferred embodiment, each of said self-assembling aggregation domains comprises, preferably consists of 3 to 5 consecutively linked repeat units, wherein said repeat units are selected of the group consisting of SEQ ID NO: 1-4, 7, 10 and 11. According to a very preferred embodiment, said self-assembling aggregation domain comprises, preferably consists of (AEAEAKAK)ₙ, wherein n is an integer from 3 to 5. According to a very preferred embodiment, said self-assembling aggregation domain comprises, preferably consists of (AEAEAKAK)ₙ, wherein n is 3 or 5.

According to a preferred embodiment, the hydrogel-forming peptide has the formula A-B-A or A-B-A-B-A, wherein A is covalently linked to the B domain, either directly or via a peptide linker, and said self-assembling aggregation domain A comprises, preferably consists of (AEAEAKAK)ₙ, wherein n is an integer from 3 to 5. Preferably, SEQ ID NO: 69 is attached to the C terminus of said hydrogel-forming peptide and/or the amino acid sequence (PRG) is inserted between the middle or C-terminal B domain and C-terminal A domain of the hydrogel-forming peptide and/or the amino acid sequence (AEF) is located between the N terminal A domain and the middle or N-terminal B domain.

### 2. Hydrophilic non-self-assembling linker domain

The hydrogel-forming protein of the invention comprises at least two hydrophilic non-self-assembling linker domains.

According to a preferred embodiment, the hydrogel-forming protein of the invention comprises 2 to 4, preferably 2 or 3, most preferably 2 hydrophilic non-self-assembling linker domains.

The term "hydrophilic non-self-assembling linker domain", "hydrophilic linker domain", "hydrophilic peptide domain" or "hydrophilic domain" used herein interchangeably refers to peptides that possess hydrophilic (Gly, Asn, Gln, Ser, homoserine, His, Thr, Cys, His) and charged (Lys, Arg, Asp, Glu) amino acid residues. The portion of hydrophilic amino acid residues included in the hydrophilic linker domain is preferably from 50% to 100%, more preferably from 60% to 100%, again more preferably from 70% to 100%, once again more preferably from 80% to 100%. The percentage of charged amino acid residues is from 8% to 100%, preferably from 8% to 30%. In another very preferred embodiment, the portion of hydrophilic amino acid residues included in the hydrophilic linker domain is from 8% to 20%.

The term "non-self-assembling domain" refers to disordered domains that do not contain any specific molecular recognition motifs, i.e. they do not assemble into supramolecular structures. The hydrophilic linker domain is typically and preferably structurally disordered, flexible, and highly water-soluble.

According to a preferred embodiment, the hydrophilic linker domain has a length of more than 30, preferably more than 33 amino acids. According to another preferred embodiment, the hydrophilic linker domain has a length from 33 to 66 amino acids. According to a more preferred embodiment, the hydrophilic linker domain has a length from 33 to 55 amino acids.

According to a preferred embodiment, the hydrophilic linker domain has a random-coil or unstructured conformation.

According to a preferred embodiment, the hydrophilic linker domain comprises, preferably consists of at least two repeat units (called herein linker repeat units to differentiate them from the repeat units of the A domain).

Said linker repeat units are oligomeric peptide motifs that are repeated within the hydrophilic linker domain, wherein the repeat units are consecutively linked, either (i) via a peptide linker or a functional oligomeric peptide stretch, such as a protease cleavage site or (ii) directly, wherein option (ii) is preferred.

According to a preferred embodiment, the hydrophilic linker domain comprises at least one linker repeat unit comprising, preferably consisting of, the sequence GXGSGSG (SEQ ID NO: 70) or GXGSGSGRGDS (SEQ ID NO: 71), in which X represents a charged amino acid such as lysine, arginine, aspartic acid or glutamic acid; or a polar amino acid, such as serine, homoserine, histidine, threonine, glutamine, or asparagine According to a more preferred embodiment, the hydrophilic linker domain comprises at least one linker repeat unit comprising the sequences of SEQ ID NO: 70 or 71, in which X is lysine or serine. According to a most preferred embodiment, X is serine. According to another preferred embodiment, X is lysine.

Preferably, the linker repeat unit is selected from the group consisting of SEQ ID NO: 21-27 and 72. According to a preferred embodiment, the hydrophilic linker domain comprises at least one linker repeat unit comprising the sequence GSGSGSG (SEQ ID NO: 21) or GKGSGSG (SEQ ID NO: 72). According to another more preferred embodiment, the hydrophilic linker domain consists of at least one linker repeat unit comprising the sequence GSGSGSG (SEQ ID NO: 21) or GKGSGSG (SEQ ID NO: 72). According to an again more preferred embodiment, the hydrophilic linker domain comprises at least one linker repeat unit comprising the sequence GSGSGSG (SEQ ID NO: 21). According to another more preferred embodiment, the hydrophilic linker domain consists of at least one linker repeat unit comprising the sequence GSGSGSG (SEQ ID NO: 21).

According to a preferred embodiment, the hydrophilic linker domain comprises 2 to 7 linker repeat units. According to a more preferred embodiment, the hydrophilic linker domain consists of 2 to 7 linker repeat units. According to a more preferred embodiment, the hydrophilic linker domain comprises of 2 to 5 linker repeat units. According to an even more preferred embodiment, the hydrophilic linker domain consists of 2 to 5 linker repeat units.

Preferably, the linker repeat unit is selected from the group consisting of SEQ ID NO: 21-27 and 72, more preferably SEQ ID NO: 21-27. In another embodiment, the linker repeat unit is selected from the group consisting of SEQ ID NO: 21-26.

| SEQ ID NO: | Sequence of linker repeat unit: |
|---|---|
| 21 | GSGSGSG |
| 72 | GKGSGSG |
| 22 | GSGSGSGSG |
| 23 | GSGSGSGRGDS |
| 24 | GSGSGSGPHSRN |
| 25 | GSGSGSGPHSRNS |
| 26 | GSGSGSGSGRGDS |
| 27 | GKGSGSGRGDS |

According to a more preferred embodiment, the hydrophilic linker domain consists of 2 to 5 linker repeat units, wherein the linker repeat unit is selected from the group consisting of SEQ ID NO: 21-27, and the repeat units are consecutively linked, either (i) via a peptide linker or a functional oligomeric peptide stretch or (ii) directly, wherein option (ii) is preferred.

In a preferred embodiment, the non-self-assembling linkers can be functionalized with one or more integrated multiple cell-binding domains. Either all of the linkers are functionalized with the same type integrated multiple cell-binding domain or with a combination of different cell-binding domains. According to a preferred embodiment, the hydrophilic linker domain comprises at least one cell-binding domain, preferably 2 to 7 cell-binding domain. Preferably, the cell-binding domain is added to at least one, preferably to all linker repeat units. Preferably, the cell-binding domain is added to the C terminus of at least one, preferably of all linker repeat units.

Preferred cell-binding domain sequences are described in Table 2. More preferably, the cell-binding domain is selected from the group consisting of RGD, RGDX (SEQ ID NO: 28), RGDS (SEQ ID NO: 29), PHSRN (SEQ ID NO: 31) and PHSRNS (SEQ ID NO: 32). Again more preferably, the cell-binding domain is selected from the group consisting of RGD, RGDS (SEQ ID NO: 29), PHSRN (SEQ ID NO: 31) and PHSRNS (SEQ ID NO: 32).

**Table 2. Preferred cell-binding domain sequences.**

| SEQ ID NO: | Peptide Sequence | Function |
|---|---|---|
| 28, 29 | RGD (no SEQ ID NO.), RGDX,RGDS | Cell adhesion sequence to Fibronectin |
| 30 | GRGDSP | Primary recognition site of α5β1 integrin |
| 31, 32 | PHSRN, PHSRNS | Synergy binding site of α5β1 integrin receptor |
| 33 | REDV | CS5 cell-binding domain to fibronectin |
| 34 | IKVAV | αLaminin motif, binding to β1 integrin |
| 35 | YIGSR | β1 Laminin motif |
| 36 | RYVVLPR | β1 Laminin motif |
| 37 | RNIAEIIKDI | γ Laminin motif |
| 38 | CKAAKRPKAAKDKQTK | Heparin-binding domain |

Preferably, the N- and/or C-terminal of the A or B domains of the hydrogel-forming protein, independently of each other, comprise at the N- and/or C terminus of the hydrogel-forming protein one or more functional oligomeric peptide stretches, such as a His-tag or the sequence GTSGS (SEQ ID NO: 69). According to one embodiment, the hydrogel-forming peptide of the invention comprises an N- or C-, preferably an N-terminal affinity tags for chromatographic purification, such as a polyhistidine tag (His-tag). Preferably, said His-tag consists of 6 histidine residues. According to another preferred embodiment, the hydrogel-forming peptide of the invention comprises an N- or C-, preferably a C-terminal sequence of SEQ ID NO: 69 (GTSGS).

According to a preferred embodiment, the hydrogel-forming peptides of the invention comprise one or more protease cleavage sites, e.g. as described in J. Patterson, J.A. Hubbell, Biomaterials 31, 7836, 2010; T. D. Sutherland, T. D. Rapson, M. G. Huson, J. S. Church, Chapter 15 in Fibrous Proteins: Structures and Mechanisms, Springer 2017. Preferably, the protease cleavage site(s) is/ are included in the hydrophilic linker domains. More preferably, said protease cleavage site(s) is/are included between two linker repeat units of the hydrophilic linker domain. Alternatively, the protease cleavage site(s) is/are included between the A and B domain. Preferred protease cleavage sites are described in Table 3.

**Table 3. Preferred protease cleavage sites sequences.**

| SEQ ID NO: | Peptide Sequence |
|---|---|
| 39 | SGESPAYYTA |
| 40 | GPQGIAGQ |
| 41 | CPQGIWGQ |
| 42 | GDQGIAGF |
| 43 | VPMSMRGG |
| 44 | VMPSMRG |
| 45 | VPMSMR |
| 46 | RPMSMR |
| 47 | IPVSLRSG |
| 48 | RPFSMIMG |
| 49 | VPLSLYSG |
| 50 | IPESLRAG |
| 51 | PAYYTA |
| 52 | YAAPVRGG |
| 53 | GTAGLIGQ |
| 54 | GMGPSGPN |
| 55 | GTARSAS |
| 56 | TSHRSAS |
| 57 | DRIRSAS |

According to a preferred embodiment, the peptide linker consists of 1-8, preferably 1-7, more preferably 1-6, again more preferably 1-5, again more preferably 1-4 consecutively linked amino acids. According to a preferred embodiment, the peptide linker comprises, preferably consists of a sequence selected from the group consisting of amino acid sequences (AEF), (DW), (PRG), SEQ ID NOs: 73 (GSGW), 74 (GSGPRG), 75 (AEFW), 69 (GTSGS), 76 (GSGS), and 77 (HHHHHHGSGS).

According to a preferred embodiment, the hydrogel-forming peptide has the formula A-B-A or A-B-A-B-A, and A is covalently linked to the B domain, either directly or via the peptide linker, wherein the hydrophilic linker domain B comprises 2 to 5 linker repeat units selected from the group consisting of SEQ ID NO: 21-27, preferably the linker repeat units are consecutively and covalently connected to each other. According to a preferred embodiment, the hydrogel-forming peptide has the formula A-B-A or A-B-A-B-A, and A is covalently linked to the B domain, either directly or via the peptide linker, wherein the hydrophilic linker domain B consists of 2 to 5 linker repeat units selected from the group consisting of SEQ ID NO: 21-27, and the linker repeat units are consecutively and covalently connected to each other. Preferably, in said hydrogel-forming peptides, SEQ ID NO: 69 is attached to the C terminus of said hydrogel-forming peptide and/or the amino acid sequence (PRG) is inserted between the middle or C-terminal B domain and C-terminal A domain of the hydrogel-forming peptide and/or the amino acid sequence (AEF) is located between the N terminal A domain and the middle or N-terminal B domain.

### 3. Rheometry

A number of single-component hydrogels from peptides and proteins with beta-sheet forming sequences have been described in the state of the art. However, these hydrogels do not achieve the rheological and mechanical properties of the hydrogel-forming protein of the invention. To consider a biomaterial suitable for extrusion-based additive manufacturing, multiple criteria have to be achieved. In particular, the material needs to have the right rheological (e.g. shear-thinning and self-healing) and mechanical (e.g. strong gel) properties.

The inventors surprisingly discovered that hydrogel-forming peptides of block co-polymers comprising at least two beta-sheet forming self-assembling aggregation domains (A domains), wherein two of the at least two A domains are interlinked by a soluble non-self-aggregating linker domains, lead to the formation of strong hydrogels with excellent rheological and biofunctional properties for biofabrication.

Rheological properties described herein were assessed using the assays described in Example 9 below. Gel formation can be assessed in a quantitative manner, e.g., using the rheological assays described herein, or in a qualitative manner, including simple visual examination.

Rheological assays can be performed to test the viscoelastic properties of the peptide scaffolds. For example, oscillatory rheometry, which subjects samples to oscillating stresses or oscillating strains, can be performed, e.g., using a controlled strain rheometer, which shears the sample at a controlled strain within a range of frequencies. Various rheometers that can be used are commercially available. Principles of rheometry and its applications to gels, are described, for example, in Clark et al., 1987 ("Structual and Mechanical Properties of Biopolymer Gels", Springer-Verlag, Berlin, 1987; Vol.83, pp.58-192 (85-86); or Kavanagh, G.M. et al. 1998 ("Rheological characterization of polymer gels," Prog. Polym. Sci. (1998) 23:533-562).

The abbreviation G' is defined herein as storage modulus which represents the elastic/solid character of the material. The abbreviation G" is defined herein as loss modulus which represents the viscous/fluid character of the material.

In oscillatory rheometry, for a viscous solution, the viscous component of the complex modulus, the loss modulus (G") typically decreases with decreasing oscillatory frequencies, and the storage modulus G' is low. For gels, G' and G" are relatively constant with oscillatory frequency. For details of the measurement, see Example 9.

### Plateau modulus

The hydrogel-forming protein of the invention, preferably formulated with concentration showed high cytocompatibility and suitable mechanical properties. For details of the measurement, see Example 9.

In a very preferred embodiment, the hydrogel-forming protein of the invention has a plateau modulus of 1-25 kPa when measured in a linear region using a dynamic frequency sweep test (ω = 100-0.1 rad s⁻¹), with a protein concentration range from 50-100 mg ml⁻¹. In another embodiment, the hydrogel-forming protein of the invention has a plateau modulus of 0.5 kPa or greater, preferably greater than 0.5 kPa (>0.5 kPa), again more preferably from >0.5 kPa to 34 kPa, when measured in a linear region using a dynamic frequency sweep test (ω = 100-0.1 rad s⁻¹), with a protein concentration range from 50-100 mg ml⁻¹. In another embodiment, the hydrogel-forming protein of the invention has a plateau modulus of 0.5 to 34 kPa, preferably 0.7 to 34 kPa, both at a protein concentration range of 18-141 mg ml⁻¹ (same measurement conditions as above). In another embodiment, the hydrogel-forming protein of the invention has a plateau modulus of 1 to 34 kPa, at a protein concentration range of 49-141 mg ml⁻¹ (same measurement conditions as above). In another embodiment, the hydrogel-forming protein of the invention has a plateau modulus of 1 to 22 kPa at a concentration range of 49-141 mg ml⁻¹ (same measurement conditions as above).

### Self-healing properties

A self-healing material or property is defined herein in that the storage modulus (G') is able to recover to >10% of initial value after high shear interval (γ = 100 %), preferably after 2 min of ω = 100 %, γ = 100 %, after 2 min of ω = 100 %, as demonstrated for the hydrogels of the invention. In a preferred embodiment, the hydrogel-forming protein of the invention is a self-healing material (i.e. recovery of G' >10% of the initial value, γ = 100 %, after 2 min of ω = 100 %), preferably for a protein of 9-10 wt%. In another preferred embodiment, G' is recovered by from >10% to 99% of the initial value, preferably for a protein of 9-10 wt%. In another preferred embodiment, G' is recovered by from >10% to 94% of the initial value, preferably for a protein of 9-10 wt%, more preferably 9 wt%. In another preferred embodiment, G' is recovered by from >10% to 91% of the initial value, preferably with a protein of 9-10 wt%, more preferably 10 wt%. For details of the measurement, see Example 9 (recovery of G' after 2 min of ω = 100 %, γ = 100 %, after 2 min of ω = 100 %).

**Shear-thinning properties** Rotational shear rate measurements were performed to evaluate viscosity changes with increasing shear rate and fitted to a Power Law model. For details of the measurement, see Example 9.

Shear-thinning parameter (n) is n<1 when the protein is shear-thinning. In a preferred embodiment, the hydrogels of the invention are shear-thinning i.e. n is <1.

In a preferred embodiment, the hydrogels of the invention self-assemble into fibrillary structures, preferably amyloid-like fibres. The hydrogels of the invention allows for to be printed in self-supporting multi-layered structures and enable cell spreading in lower concentration gels of 45 and 65 mg mL⁻¹.

According to a preferred embodiment, the hydrogel-forming peptide has the formula A-B-A or A-B-A-B-A, wherein A is the self-assembling aggregation domain covalently linked to the hydrophilic non-self-assembling linker domain B domain, either directly or via a peptide linker; the self-assembling aggregation domain A comprises the sequence (AEAEAKAK)ₙ, wherein n is an integer from 3 to 5; and the hydrophilic linker domain B comprises 2 to 5 linker repeat units, wherein the linker repeat unit is selected from the group consisting of SEQ ID NO: 21-27, preferably the linker repeat units are consecutively and covalently connected to each other. According to another preferred embodiment, the hydrogel-forming peptide has the formula A-B-A or A-B-A-B-A, wherein A is covalently linked to the B domain, either directly or via a peptide linker; the self-assembling aggregation domain A consists of (AEAEAKAK)ₙ, wherein n is an integer from 3 to 5; and the hydrophilic linker domain B consists of 2 to 5 linker repeat units, wherein the linker repeat unit is selected from the group consisting of SEQ ID NO: 21-27, preferably the linker repeat units are consecutively and covalently connected to each other. Preferably, in said hydrogel-forming peptides, SEQ ID NO: 69 is attached to the C terminus of said hydrogel-forming peptide and/or the amino acid sequence (PRG) is inserted between the middle or C-terminal B domain and C-terminal A domain of the hydrogel-forming peptide and/or the amino acid sequence (AEF) is located between the N terminal A domain and the middle or N-terminal B domain

According to a preferred embodiment, the hydrogel-forming peptide of the invention has a sequence selected from the group consisting of SEQ ID NO: 58-65 (Table 7).

**Table 7.**

| SEQ ID NO: | Sequence name | Sequence of hydrogel-forming peptide: |
|---|---|---|
| 58 | PB1 | HHHHHHGSGS-(AEAEAKAK)5-AEF-(GSGSGSGRGDS)5-W- (AEAEAKAK)5-DW-(GSGSGSGRGDS)5-PRG-(AEAEAKAK)5- GTSGS |
| 59 | PB2-H-tr | HHHHHHGSGS-(AEAEAKAK)5-AEF-(GSGSGSGPHSRN- SGSGSGSGSGRGDS)2-GSGW-(AEAEAKAK)3-DW- GSGSGSGRGDS-GSGSGSGPHSRNS-GSGSGSGSGRGDS- GSGPRG-(AEAEAKAK)5-GTSGS |
| 60 | PB2-H | HHHHHHGSGS-(AEAEAKAK)5-AEF-(GSGSGSGPHSRN- SGSGSGSGSGRGDS)2-GSGW-(AEAEAKAK)5-DW- (GSGSGSGPHSRNS-GSGSGSGSGRGDS)2-GSGPRG- (AEAEAKAK)5-GTSGS |
| 61 | PB2-H NoHis | (AEAEAKAK)5-AEF-(GSGSGSGPHSRN-SGSGSGSGSGRGDS)2- GSGW-(AEAEAKAK)5-DW-(GSGSGSGPHSRN- SGSGSGSGSGRGDS)2-GSGPRG-(AEAEAKAK)5-GTSGS |
| 62 | PB2-H-TR NoHis | (AEAEAKAK)5-AEF-(GSGSGSGPHSRNSGSGSGSGRGDS)2- GSGW-(AEAEAKAK)3-DW-GSGSGSGRGDS-GSGSGSGPHSRNS- GSGSGSGSGRGDS-GSGPRG-(AEAEAKAK)5-GTSGS |
| 63 | MMP-PB2 | MRGSHHHHHHGSGS-(AEAEAKAK)5-AEF-GSGSGSGRGDS- GSGESPAYYTA-(GSGSGSGRGDS)3-W-(AEAEAKAK)5-DW- |
| | | (GSGSGSGRGDS)5-PRG-(AEAEAKAK)5-GTSGS |
| 64 | PB2-K1 | MRGSHHHHHHGSGS-(AEAEAKAK)5-AEF-GKGSGSGRGDS- (GSGSGSGRGDS)4-W-(AEAEAKAK)5-DW-(GSGSGSGRGDS)5- PRG-(AEAEAKAK)5-GTSGS |
| 65 | PB2-K2 | MRGSHHHHHHGSGS-(AEAEAKAK)5-AEF-GKGSGSGRGDS- (GSGSGSGRGDS)4-W-(AEAEAKAK)5-DW-GKGSGSGRGDS- (GSGSGSGRGDS)4-PRG-(AEAEAKAK)5-GTSGS |

According to a very preferred embodiment, the hydrogel-forming peptide of the invention having the formula A-B-A has a sequence selected from the group consisting of SEQ ID NO: 66-68 (Table 8).

**Table 8.**

| SEQ ID NO: | Sequence name | Sequence of hydrogel-forming peptide: |
|---|---|---|
| 66 | PB0 | HHHHHHGSGS-(AEAEAKAK)5-AEF-(GSGSGSGRGDS)7- PRG-(AEAEAKAK)5-GTSGS |
| 67 | PB0-IncW | HHHHHHGSGS-(AEAEAKAK)5-AEFW-(GSGSGSGRGDS)7- PRG-(AEAEAKAK)5-GTSGS |
| 68 | PB0 NoHis | (AEAEAKAK)5-AEF-(GSGSGSGRGDS)7-PRG-(AEAEAKAK)5- GTSGS |

According to another preferred embodiment, the hydrogel-forming peptide of the invention has a sequence selected from the group consisting of SEQ ID NO: 58-68.

In a further aspect, the invention relates to a protein comprising a hydrophilic non-self-assembling linker domain (domain B) comprising at least one linker repeat unit comprising, preferably consisting of, the sequence GXGSGSG (SEQ ID NO: 70) or GXGSGSGRGDS (SEQ ID NO: 71), in which X represents a charged amino acid such as lysine, arginine, aspartic acid or glutamic acid; or a polar amino acid, such as serine, homoserine, histidine, threonine, glutamine, or asparagine.

According to a more preferred embodiment, the hydrophilic linker domain comprises at least one linker repeat unit comprising the sequences of SEQ ID NO: 70 or 71, in which X is lysine or serine. According to a most preferred embodiment, X is serine. According to another preferred embodiment, X is lysine. Preferably, said protein is a hydrogel-forming protein. Disclosure and embodiments described under items 1., 2. and 3. above, especially item 2., also apply to this aspect.

According to a preferred embodiment, the hydrophilic linker domain comprises, preferably consists of at least one linker repeat unit.

According to a preferred embodiment, the hydrophilic linker domain comprises at least one linker repeat unit comprising the sequence GSGSGSG (SEQ ID NO: 21) or GKGSGSG (SEQ ID NO: 72).

According to another more preferred embodiment, the hydrophilic linker domain consists of at least one linker repeat unit comprising the sequence GSGSGSG (SEQ ID NO: 21) or GKGSGSG (SEQ ID NO: 72).

According to an again more preferred embodiment, the hydrophilic linker domain comprises at least one linker repeat unit comprising the sequence GSGSGSG (SEQ ID NO: 21). According to another more preferred embodiment, the hydrophilic linker domain consists of at least one linker repeat unit comprising the sequence GSGSGSG (SEQ ID NO: 21).

According to another preferred embodiment, the hydrophilic linker domain comprises at least one linker repeat unit comprising the sequence of SEQ ID NO: 27. According to another more preferred embodiment, the hydrophilic linker domain consists of at least one linker repeat unit comprising the sequence of SEQ ID NO: 27.

According to a preferred embodiment, the hydrophilic linker domain has a length of more than 30, preferably more than 33 amino acids. According to another preferred embodiment, the hydrophilic linker domain has a length from 33 to 66 amino acids. According to a more preferred embodiment, the hydrophilic linker domain has a length from 33 to 55 amino acids.

Said linker repeat units are oligomeric peptide motifs that are repeated within the hydrophilic linker domain, wherein the repeat units are consecutively linked, either (i) via a peptide linker or a functional oligomeric peptide stretch or (ii) directly, wherein option (ii) is preferred.

Preferably, the sequence of the linker repeat unit comprises, preferably consists of SEQ ID NO: 70 or 71. Preferably the sequence of the linker repeat unit is selected from the group consisting of SEQ ID NO: 21-27and 72, more preferably of SEQ ID NO: 22-27 or 21-26.

According to a more preferred embodiment, the hydrophilic linker domain consists of 2 to 5 linker repeat units, wherein the linker repeat unit is selected from the group consisting of SEQ ID NO: 22-27, and the repeat units are consecutively linked, either (i) via a peptide linker or a functional oligomeric peptide stretch or (ii) directly, wherein option (ii) is preferred. According to a preferred embodiment, the block-copolymer protein of the invention comprises 1 to 4, preferably 1 or 3, most preferably 1 or 2 hydrophilic non-self-assembling linker domains.

According to a preferred embodiment, the hydrophilic linker domain has a length of more than 30, preferably more than 33 amino acids. According to another preferred embodiment, the hydrophilic linker domain has a length from 33 to 66 amino acids. According to a more preferred embodiment, the hydrogel-forming protein of the invention the hydrophilic linker domain has a length from 33 to 55 amino acids.

According to a preferred embodiment, the hydrophilic linker domain comprises of 2 to 7 linker repeat units. According to a more preferred embodiment, the hydrophilic linker domain consists of 2 to 7 linker repeat units. According to a preferred embodiment, the hydrophilic linker domain comprises of 2 to 5 linker repeat units. According to a more preferred embodiment, the hydrophilic linker domain consists of 2 to 5 linker repeat units.

According to one embodiment, the B domain in the block-copolymer protein comprises one or several bioactive sites, selected from the group consisting of cell-binding domains, protein-binding domains, protease-cleavage sites, growth-factor-binding sites, sequences with anti-microbial activities, sequences with metal-binding activities, sequences with mineralization activities and sequences with growth factor activities.

In a preferred embodiment, the non-self-assembling linkers can be functionalized with one or more integrated multiple cell-binding domains. Either all of the linkers are functionalized with the same type integrated multiple cell-binding domain or with a combination of different cell-binding domains. According to a preferred embodiment, the hydrophilic linker domain comprises at least one cell-binding domain, preferably 2 to 7 cell-binding domain. Preferably, the cell-binding domain is added to at least one, preferably to all linker repeat units. Preferably, the cell-binding domain is added to the C terminus of at least one, preferably of all linker repeat units. Preferably, the cell-binding domain is selected from the group consisting of RGD (SEQ ID NO: 28), RGDS (SEQ ID NO: 29), PHSRN (SEQ ID NO: 31) and PHSRNS (SEQ ID NO: 32).

According to one embodiment, the B domain in the block-copolymer protein comprises two or more cell-binding domains. Preferred cell-binding domain sequences are described in **Table 2.**

According to one embodiment, the non-self-assembling linker B in a block-copolymer protein comprises a cell binding domains comprising the sequences RGD and PHSRN.

In another embodiment, the non-self-assembling linker B in a block-copolymer protein comprise a combination of bioactive sites with different biological functions. According to one embodiment, the non-self-assembling linker B in a block-copolymer protein comprise a combination of cell-binding domains and protease-cleavage sites. Preferred protease-cleavage sequences are described in **Table 3**. According to one embodiment, the non-self-assembling linker B in a block-copolymer protein comprise combinations of cell binding domains with RGD sequences and SGESPAYYTA protease-cleavage sites.

### Methods for preparing the hydrogel-forming peptides and 3D biofabrication

In another aspect, the invention refers to a method for preparing the hydrogel-forming peptide of the present invention. The novel proteins can be produced via chemical synthesis or genetic recombination, using state-of-the-art microbial expression systems and purification processes. Preferably, the hydrogel-forming peptides of the present invention are prepared via genetic recombination, using state-of-the-art microbial expression systems and purification processes.

In another aspect, the invention refers to a method for 3D biofabrication using the hydrogel-forming peptide of the present invention.

Biofabrication is herein broadly defined to refer to fabrication of biologically functional products through, e.g., bioprinting or bioassembly. The term includes spatial patterning of living cells and other biologics by stacking and assembling them using a computer-aided layer-by-layer deposition approach for fabrication of living tissue and organ analogs organ printing; computer-aided 3D printing technologies, using layer-by-layer deposition of cells and/or cell aggregates into a 3D gel with sequential maturation of the printed construct into tissue or organs; material transfer processes for patterning and assembling biologically relevant materials-molecules, cells, tissues, and biodegradable biomaterials; computer-aided transfer processes for patterning and assembling living and non-living materials with a prescribed 2D or 3D organization; production of bioengineered structures serving, e.g., in regenerative medicine, pharmacokinetic and basic cell biology studies; production of complex living and non-living biological products from raw materials, such as living cells, molecules, extracellular matrices, and biomaterials; Additive Manufacturing, e.g. extrusion-based additive manufacturing (cf. also Groll et al., Biofabrication 8, 2016, 013001); positioning of cells at defined coordinates in 2D or 3D using automated and computer controlled techniques (Moroni et al., 2018, Trends Biotechnol. 36, pp. 384-402), usually with the aid of biomaterials that are either (i) directly processed with the cells as suspensions/dispersions, (ii) deposited simultaneously in a separate printing process, or (iii) used as a transient support material.

In another aspect, the invention refers to a bioink comprising the hydrogel-forming peptide of the invention. In another aspect, the invention refers to use of the hydrogel-forming peptide of the invention as bioink, preferably for three-dimensional (3D) biofabrication. Bioinks are defined herein as materials with rheological and biofunctional properties to enable biofabrication. Bioinks include biologically active components or molecules, with and without cells.

In a preferred embodiment, the hydrogel-forming protein of the invention or the ink of the invention is formulated with a protein concentration between 50-100 mg ml-1.

The invention will now be illustrated by the following non-limiting examples.

### EXAMPLES

DNA sequences of the protein were designed for expression in *E. coli.* His-tag was used for protein purification. Protein self-assembly into beta-sheet was confirmed using FTIR (Perkin-Elmer SpectrumTwo) and amyloid-like structures were observed with TEM (FEI Morgagni 268) and SEM (JOEL JSM 7100F). Shear rheometry (Anton-Paar MCR 502) was used to characterize protein thermal gelation process as well as final hydrogel viscoelastic properties. To confirm material cytocompatibility, we performed 2D human mesenchymal stem cell (hMSC) seeding as well as 3D hMSC encapsulation using a range of hydrogel concentrations and mechanical properties. Fluorescent microscopy was used to evaluate cell viability and spreading. Finally, cell-laden bioinks were processed via biofabrication (Cellink BioX) to find suitable printing parameters and evaluate material applicability for tissue engineering.

### Example 1 - Molecular Cloning

DNA sequences of the hydrogel-forming proteins of the invention were optimized for expression in E. coli and in some cases optimized using a codon scrambler for repetitive sequences (http://chilkotilab.oit.duke.edu/static/).

DNA fragments encoding the genes of the hydrogel-forming protein of the invention were then produced by assembly polymerase chain reaction (PCR) of shorter fragments. All oligonucleotides used for cloning were purchased from Microsynth AG. Assembly PCR was carried out in two steps. In both cases Phusion polymerase (New England Biolabs) was used following the recommended protocols, with equimolar mixtures of fragments providing a total of 100-200 ng of template DNA. For the first assembly PCR step, initial denaturation at 98 °C for 30 s was followed by 30 cycles comprising 98 °C for 2 s, 60 °C for 15 s and 72 °C for 35 s; a final extension of 5 minutes was used. For the second assembly PCR step, the amplifying primers and additional dNTPs were added and the same thermocycle program employed. PCR products were purified by 1.5 % agarose gel, the bands cut out with reference to a DNA ladder (100 bp DNA Ladder, New England Biolabs) and isolated using a DNA gel extraction kit (Zymo Research).

The purified PCR products were cloned into the vector pET29b(+) through restriction site ligation. In a typical workflow, full-length PCR products and pET29b(+) plasmid containing a stuffer fragment between restriction sites were digested with *BamHI*/*Spel* or *EcoRI*/*SacII* using the supplier's (New England Biolabs) recommended protocol. Additionally, the vector was supplied with shrimp alkaline phosphatase (rSAP, New England Biolabs) during digestion to remove the terminal phosphate groups, preventing self-ligation. The digested products were purified using a DNA Clean & Concentrator kit (Zymo research). Ligation of the digested insert and digested plasmid backbone was performed using T4 DNA ligase (New England Biolabs) and 10 µL of the ligation reaction was used to transform chemically-competent *E. coli* strain XL1-Blue (Agilent) using heat shock. Successfully transformed clones were selected on (LB) agar plates containing 100 µg mL⁻¹ kanamycin sulfate (PanReac AppliChem). Single colonies of transformants were grown in LB pre-cultures containing 50 µg mL⁻¹ kanamycin sulfate at 37 °C overnight and harvested by centrifugation. Plasmids were isolated using a ZR Plasmid Miniprep-Classic kit (Zymo Research). Plasmid sequences were confirmed by Sanger DNA sequencing by Microsynth AG (Switzerland), using the T7 primer.

To prepare PB0-incW and MMP-PB2, the QuikChange^{®} (Agilent Technologies) method was used, which introduces point mutations using PCR. Primers were designed with the QuikChange^{®} Primer Design program (Agilent Technologies). Each primer was mixed with its corresponding template, PB0 for PB0-incW and PB1 for MMP-PB2. The PCR reactions were carried out using Phusion polymerase (New England Biolabs) according to the manufacturers standard protocol, using 20 cycles of amplification with annealing temperature adjusted for each primer set and an elongation time of 2.5 minutes to cover the entire plasmid. To digest the templates, reaction mixtures were treated with Dpn1 (New England BioLabs) for 2 hours of incubation at 37 °C. The target plasmids were purified with DNA Clean & Concentrator Kits (Zymo Research) and used for transformation into E. coli strain XL1-Blue as described above.

### Example 2 - Heat shock transformation of competent E. coli cells

The method has been adapted from Froger A, Hall JE. Transformation of plasmid DNA into E. coli using the heat shock method (J Vis Exp. 2007 (6):253. doi:10.3791/253). To 50 µL of competent *E. coli* BI21 (DE3) cells thawed on ice, 0.5-5 µL of plasmid DNA (approximately 40 ng µL⁻¹) was added and the mixture incubated on ice for 15-30 min. Heat shock was induced by placing the transformation mixture in a 42 °C heat block for 20-30 sec. After a five-minute incubation period on ice, 250 µL of pre-warmed LB medium was added and the cells were shaken for one or two hours at 37 °C and 180 rpm to induce antibiotic resistance. To select the transformed cells, 100 to 300 µL of the cell suspension was plated out on LB plates with the appropriate antibiotics and incubated overnight at 37 °C in an incubator.

### Example 3 - Screening of Expression conditions

For assessment of suitable expression and purification condition, expression strength of the new designed proteins was screened using different media and expression temperatures. The different experiments indicated that all proteins of the invention (SEQ ID NO: 59-68) were soluble expressible at 30°C in TB media.

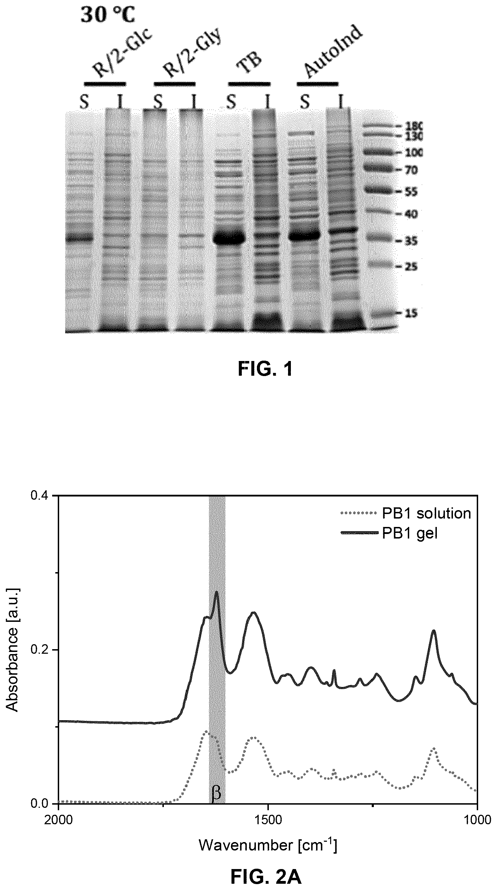

In Figure 1, the results for PB1 are presented with OD normed samples of the soluble and insoluble fractions after cultivation in different media. The greatest amount of soluble protein could be detected by cultivation in TB media. This also applies to the further hydrogel-forming protein of the invention (SEQ ID NO: 59-68).

*E. coli* strains were cultured routinely in lysogeny broth (LB) or terrific broth (TB) and, if necessary, were supplemented with kanamycin (0.05 mg mL⁻¹). TB medium consisting of 24 g L⁻¹ of yeast extract, 12 g L⁻¹ of tryptone and 4 g L⁻¹ of glycerol and a separately prepared 1:10 saline solution (0.72 M on KH₂PO₄ and 0.17 M on KH₂PO₄). Subsequently, 100 mL of the saline solution is added to 900 mL of the sterile TB medium. LB medium consisting of 5 g L⁻¹ of yeast extract, 10 g L⁻¹ of tryptone and 10 g L⁻¹ of NaCl. For expression screening also R/2 medium (pH 6.8)(Seong, H.J., Woo, J.E. & Jang, YS. Sci Rep 10, 12132 (2020). https://doi.org/10.1038/s41598-020-69143-3) contained 2 g (NH₄)₂HPO₄, 6.75 g KH₂PO₄, 0.7 g MgSO₄·7H₂O, 0.85 g citric acid, and 5 mL trace metal solution per liter and 20 g L⁻¹ of the desired carbon source (R/2-Glc = R/2 minimal medium with glucose as carbon source, R/2-Gly = R/2 minimal medium with glycerol as carbon source) was used. The trace metal solution contained (per liter): 0.1 M HCI, 10 g FeSO₄·7H₂O, 2.25 g ZnSO₄·7H₂O, 0.58 g MnSO₄·5H₂O, 1 g CuSO₄·5H₂O, 0.1 g (NH₄)₆Mo₇O₂₄·4H₂O, 0.02 g Na₂B₄O₇·10H₂O, and 2 g CaCl₂·2H₂O. Also the autoinduction media ZYP5052 from Studier (Protein Expr. Purif. 41, 207-234 (2005)) has been tested. Bacterial cultures were incubated in baffled Erlenmeyer flasks in a New Brunswick Innova 42 orbital shaker at 180 rpm and 37 °C. Bacteria on agar plates were incubated in a HERATherm Thermo Scientific incubator in air at 37°C. All materials and biotransformation media were sterilized by autoclaving at 121°C for 20 min. Aqueous stock solutions were sterilized by filtration through 0.20 µm syringe filters. Agar plates were prepared by using LB supplemented with 1.5 % (w/v) agar. For the expression of the protein a 5 mL preculture were cultivated for about 16 hours at 37° C and 180 rpm. From this culture, 4 mL are then removed and inoculated to baffled Erlenmeyer flasks containing 400 mL media. The expression started with 37°C at 180 rpm until the OD of 0.6 was reached then all cultures was induced with 0.1 mM IPTG with exception of the autoinduction media and turned for the next 16 h to 20 °C, 30 °C or 37 °C.

After the expression phase, cells were harvested by centrifugation for 15 min at 4500 g and 4 °C and used directly, or cell pellets were stored at -20 °C.

### Example 4 - Fermentation

For the expression of the different proteins a preculture were first in 2 L Erlenmeyer flasks (baffled), which are filled with 400 mL of TB medium, for about 16 hours at 37 ° C and 180 rpm cultured. From this culture, 400 mL are then removed and inoculated the bioreactor containing 30 L TB medium (as described above). Bioreactor cultures were performed in 50 L round bottomed stirred fermenter (B. Braun Biotech International UD 50 Biostat). The fermenter was fitted with pH and dissolved oxygen sensors (Mettler Toledo, OH, USA). Temperature was controlled via a water-filled stainless-steel base. Dissolved oxygen (DO) was controlled at 35 % of air saturation by using a sequential cascade of agitation between 250 and 800 rpm and aeration between 2 and 30 L min⁻¹ with pressure air. The pH was controlled at 7.0 using 1 % phosphoric acid and 1 M sodium hydroxide. The culture is grown at 37 °C until an OD of 0.6 was reached then the protein expression was induced by adding isopropyl-β-d-thiogalactopyranoside (IPTG 0.1 mM end concentration) before the temperature was change to 30 °C. Subsequently, culture is continued for at least 16-20 hours during the expression phase. After the expression phase cells were harvested by centrifugation for 15 min at 4500 g and 4 °C and used directly, or cell pellets were stored at -20 °C.

### Example 5 - Purification by Ni-NTA Chromatography

The proteins PB0, PB0-IncW, PB1, PB2-H-tr, PB2-H, PB2-K1, PB2-K2 and MMP-PB2 could be purified by Ni-NTA chromatography and dialysis against 2 % acetic acid.

For cell disruption, the cell biomass of 30 g was resuspended in buffer (50 mL; 20 mM TRIS, 100 mM NaCl, 8 M Urea, 5 % glycerol pH 8). Cells were disrupted by a single passage through a French pressure cell at 2000 psi. Cell debris was separated from the crude cell extract by centrifugation at 3400 g for 30 min. Purification was performed by affinity chromatography with a His tag on an automated Äkta purifier system. A 5 mL HisTrap FF column was equilibrated with buffer (50 mL; 20 mM TRIS, 100 mM NaCl, 8 M Urea, 5 % glycerol), flow rate 2.5 mL min⁻¹. After the crude cell extract had been applied to the column, unbound protein was eluted with three column volumes (CV) of buffer supplied with imidazole (15 mM). The desired enzyme was eluted with 3 CV of buffer containing imidazole (300 mM). Washing, flow through, and elution fractions were analysed by SDS-PAGE. The desired fractions were collected for dialysis (membrane cut off 10 kDa) against 2 % acetic acid. Afterwards the protein solution was centrifuged again to remove precipitations. Then the protein solution was concentrated by Amicon filters with cutoff 10 kDa to around 50 mg mL⁻¹. The protein content of the crude cell extracts as well as of the purified and desalted fractions was determined by using the BCA kit (Thermo Fisher Scientific). NanoDrop was used to determine the protein content of purified protein samples based on the corresponding extinction coefficients.

MALDI results of PB2-H-tr, PB1 and MMP9-PB2 were evaluated via SDS-Page of the purified proteins. The resulting protein masses are 21504 Da for PB2-H-tr, 24197 Da for B1 and 24330 Da MMP9-PB2. The theoretical protein masses are 21.44 kDa for PB2-H-tr, 24.13 kDa for B1 and 24.29 kDa for MMP9-PB2 . Thus, the results validate the expected protein masses.

### Example 6 - Purification without His-tag

For PB2-H-tr NoHis and PB2-H NoHis, a purification route without Ni-NTA chromatographic has been developed.

For cell disruption, the cell biomass of 30 g was resuspended in 90 mL lysis buffer (20 mM Tris, 100 mM NaCl, 5 % (w/w) Glycerol; pH 8.0). After 2 min sonication with 50 % Amplitude RNAs, DNAs and 1 mg mL⁻¹ Lysozyme were added. The solution has been incubated for 6 or 24 h at room temperature and then centrifugated for 10 min at 3400 g. Afterwards 8 M Urea were added to the supernatant and the supernatant protein solution was dialyzed as already described against 2 % acetic acid. Then the protein solution was concentrated by Amicons with cutoff 10 kDA to around 50 mg mL⁻¹. NanoDrop was used to determine the protein content of purified protein samples based on the corresponding extinction coefficients.

MALDI results of PB2-H-tr NoHis and PB2-H NoHis were evaluated via SDS-Page of the purified proteins. The resulting protein masses are 23390 Da for PB2-H-tr NoHis and 20482 Da for PB2-H NoHis. The theoretical protein masses are 23.38 kDa for PB2-H-tr NoHis and 20.47 kDa PB2-H NoHis. Thus, the results validate the expected protein masses.

### Example 7 - Gelation studies of engineered protein-based bioinks

Gel samples were prepared from protein solutions of SEQ ID NO: 58-68 in 2 % acetic acid with protein concentrations of 10-130 mg mL⁻¹. The protein concentration was adjusted using ultracentrifugation (Amicon Ultra-0.5 mL Centrifugal Filters, Merck) at 14,000 g for 2-15 min. A protein sample (1:100 dilution) was taken for protein concentration analysis (Pierce BCA Protein Assay Kit, Thermo Scientific) at this stage. The samples were then transferred to dialysis units (Micro Float-A-Lyzer, MWCO=0.5-1.0 kDa, Repligen) and placed in a PEG (Mₙ -8000, BioUltra, Sigma) solution in milliQ water to match the osmotic pressure of the protein solution. The dialysis solution was changed twice every 12 h. Then, the temperature of the dialysis bath was increased to 55 °C for 1 h to induce gelation of the protein. The process was irreversible and the gel remained stable once cooled to room temperature. The dialysis solution was changed two more times (right after gelation and after 12 h), first to PEG in milliQ water solution and then to PEG in 10 mM HEPES buffer pH 7.4 solution. This final step was used to increase the pH of the final gel.

The pH of the final gel did not completely match the one of HEPES buffer and depended on the protein type used (extract of the data):

| **Protein** | **pl** | **Mw, kDa** | **Gel pH** |
|---|---|---|---|
| PB0 | 7.94 | 16.9 | 6 |
| PB1 | 7.05 | 24.1 | 6.3-6.5 |
| PB2-H-tr | 8.92 | 21.4 | 6.5-7 |
| PB2-H | 9.05 | 24.3 | 6.5-7 |
| MMP2-PB2 | 6.64 | 24.3 | 5.5-6 |

After manual extrusion into wells of a 24-well plate, formed gels maintained shape and did not dissolve during incubation at room temperature. In addition, gel shape was maintained at 37 °C in phosphate buffer saline (PBS) and Dulbecco's Modified Eagle Medium (DMEM).

### Example 8 - Gel structure

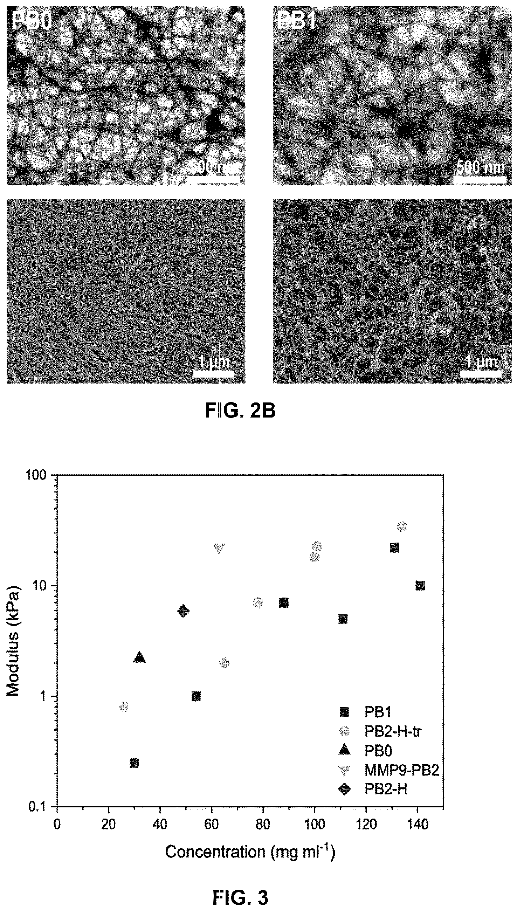

We investigated the physicochemical nature of the materials following gelation. Solutions of the proteins of SEQ ID NO: 58-68 (35 mg mL⁻¹) were dialysed against 3 wt% PEG solution in water (bath changed twice every 12 h) to prepare -150 µL of 18 mg mL⁻¹ protein solution. Half of the sample volume (-75 µL) was gelled at 60 °C for 1 h, whereas the other half was not gelled and left in the liquid-like state at 25 °C. Both types of samples (gelled and non-gelled solution) were frozen (-80 °C) and lyophilised. ATR-FTIR (Spectrum Two, Perkin Elmer) was performed on both samples to compare the protein structures (**Figure 2**).

For PB 1, a peak at 1622 cm⁻¹, indicative of beta-sheet structures, was observed in the lyophilised gel sample, whereas a peak at 1648 cm⁻¹ indicating amorphous structures was more prominent in the non-gelled sample. Comparable results were achieved for the further hydrogel-forming protein of the invention (SEQ ID NO: 59-68).

Self-assembly of the proteins into fibrillary structures was confirmed by TEM (FEI Morgagni 268) and SEM (JOEL JSM 7100F) imaging of PB0 and PB1 proteins after processing them using gelation protocol described in example 7.

### Example 9 - Rheological properties of engineered protein-based bioinks

Rheological characterisation was performed on a strain-controlled shear rheometer (MCR 502; Anton Paar) equipped with a Peltier stage with an 8 mm sandblasted plate-plate geometry and a gap size of 0.3-0.5 mm. All measurements were performed at 25°C. Oscillatory strain amplitude sweeps (shear strain range γ = 0.1-100 %, oscillatory frequency ω = 10 rad s⁻¹) were performed to measure linear viscoelastic response range (LVR) for storage (G') and loss (G") moduli. It was used to choose optimal shear strain for further measurements. Storage (G') and loss (G") moduli to evaluate solid-like and liquid-like material properties respectively were then measured by oscillatory frequency sweeps (γ = 0.3 % within LVR, ω = 100-0.1 rad s⁻¹).

Using the results of these measurements, plateau storage modulus in the range ω = 100-0.1 rad s⁻¹ was used to define and compare different protein hydrogel formulations. Rotational shear rate measurements (shear rate range δγ/δt = 0.01-100 s⁻¹) were performed to evaluate viscosity changes with increasing shear rate and fitted to a Power Law model (where viscosity η = K (δγ/δt)⁽ⁿ⁻¹⁾, and K and n are fitting parameters) to obtain shear-thinning parameter (n), where when n<1 the material is shear-thinning. The former effect is also observed as viscosity decrease upon increasing shear rate.

Step strain measurements (stepping between strains of γ = 0.3 % (within LVR) and γ = 100 % (beyond LVR, breaking of the material), ω = 10 rad s⁻¹)were used to record changes in storage (G') and loss (G") moduli upon changing strain and evaluate material self-healing.

Rheological tests were performed using PB0, PB1, PB2-H-tr, PB2-H, and MMP2-PB2 samples. The linear dependence of plateau storage modulus (G') on PB1 and PB2-H-tr protein concentration obtained via oscillatory frequency sweeps is summarised in **Figure 3** and **Table 4.** The PB0, PB1, PB2-H-tr, PB2-H, and MMP2-PB2 proteins in a concentration range of 18-141 mg ml⁻¹ could form hydrogels with plateau storage modulus of about 1 and 34 kPa. The hydrogels of the invention showed preferred mechanical properties with a plateau modulus of 1 to 25 kPa at a concentration range of 30-100 mg mL⁻¹. These values are beneficial for extrusion-based 3D printing.

A self-healing material is defined herein in that the storage modulus (G') is able to recover to >10% of initial value after high shear interval (γ = 100 %), as demonstrated for the hydrogels of the invention. For 9 wt% PB1 gel after 2 min of ω = 100 % the storage modulus (G') is recovered by 94%. For 10 wt% PB2-H-tr gel after 2 min of ω = 100 % the storage modulus (G') is recovered by 91%.

Rheological measurements were also performed and compared on formulations with and without cells (2 × 10⁶ cells mL⁻¹) on 5 wt% PB1 gels. The addition of cells had no impact on the self-healing (after 2 min of ω = 100 % the storage modulus (G') is recovered by 38 % without cells and by 31 % with cells of initial values) and shear-thinning properties (shear-thinning parameter (n) from Power Law model without cells was n = 0.13, with cells was n = 0.12) of the hydrogel **(****Figure 4****)** and only a minimal reduction on the plateau modulus (G'_{cells} is about3.9 kPa, vs. G'_{no cells} is about 4.9 kPa; Δ G' = 1 kPa).

**Table 4.**

| **Protein** | **Concentration, mg ml⁻¹** | **Storage modulus, kPa** |
|---|---|---|
| PB0 | 18 | 0.7 |
| | 120 | 9 |
| PB1 | 54 | 1 |
| | 111 | 5 |
| | 141 | 10 |
| PB2-H-tr | 26 | 0.8 |
| | 49 | 6 |
| | 100 | 18 |
| | 134 | 34 |
| PB2 | 49 | 6 |
| MMP9-PB2 | 63 | 22 |

### Example 10 - Assessment of injectability and printing parameters of protein-based bioinks

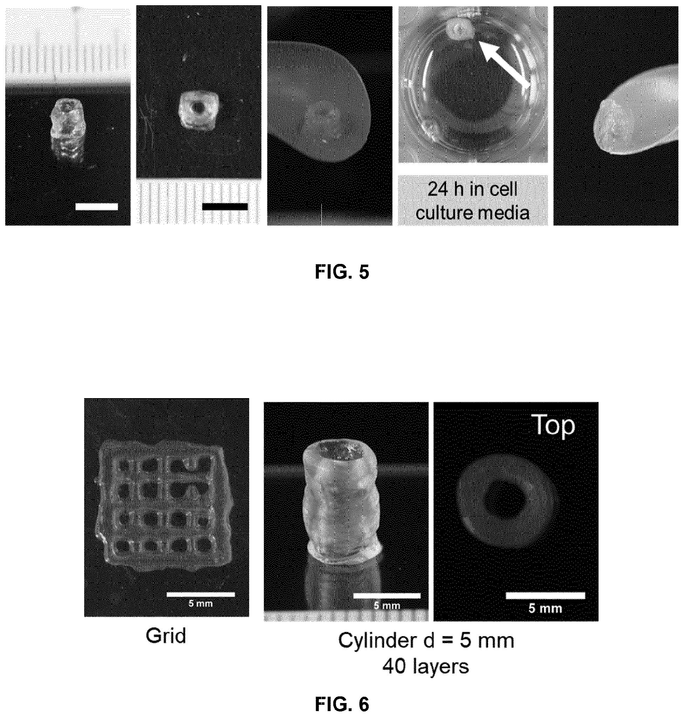

The extrudability of hydrogels based on proteins of SEQ ID NO: 58-68 was tested by gels of different formulations with hand-extrusion through 30GA needle and positive displacement pipette (**Figure 5**). The formed structures were found stable during manual manipulation.

Two protein architectures, A-B-A (PB0, PB0-IncW and PB0 NoHis) and A-B-A-B-A (PB1, PB2-H-tr, PB2-H, PB2-H NoHis, PB2-H-tr NoHis, MMP-PB2, PB2-K1, PB2-K2) were compared in terms of their self-assembly capacity and final gel properties. No significant differences were found in the TEM images of amyloid-like fibres. Slightly higher mechanical properties of the hydrogels were observed for A-B-A-B-A proteins compared to A-B-A at the same concentration.

The formulated protein hydrogels of the invention showed beneficial mechanical properties. BIO X pneumatic-driven robotic dispenser (CELLINK) was used to print 5-10 % (w/v) protein bioinks (25G nozzle; Ø 250 µm; v = 3-4 mm s⁻¹; P = 35-75 kPa) into planar (grid) and 3D structures (cylinder, total of 40 layers). The protein hydrogels, formulated with concentration between 50-100 mg ml-1, had suitable mechanical properties (i.e., plateau modulus of 1-25 kPa and a high degree of shear-thinning) for extrusion-based 3D printing. The material could be printed in self-supporting multi-layered (40 layers) structures (Figure 6).

Thus, it could be demonstrated that the hydrogels of the invention are able to print many vertical layers which is indicative of the apparent yield stress of the material that enables biofabrication.

### Example 11 - Cell types and cultivation

Three types of cells were used to test cell compatibility of protein hydrogels of the invention (SEQ ID NO: 58-68):

| **Cells** | **Cell medium used** |
|---|---|
| mouse fibroblast cell line NIH/3T3 (supplier: ATCC) | high glucose DMEM media, 10 % bovine serum, 1 % Pen Strep |
| mouse myoblast cell line C2C12 | high glucose DMEM media, 10 % fetal bovine serum, 1 % Pen Strep, 1 % Glutamax (100x), 1% Non-Essential Amino Acids, 0.05 % betamercaptoethanol |
| human mesenchymal stem cells (hMSCs) | low glucose DMEM media, 10 % fetal bovine serum, 1 % Pen Strep, fibroblast growth factor basic 0.25 mg mL⁻¹ |

### Example 12 - Cell staining

LIVE/DEAD^{™} Viability/Cytotoxicity Kit (Invitrogen, ThermoFisher) was used according to manufacturer's instructions. Samples stained for 45 min at 37 °C and then washed three times with HBSS. Fluorescent images were taken with EVOS microscope (ThermoFisher).

For actin filament staining the samples were fixed with buffered 4 % formaldehyde solution (Sigma-Aldrich) for 1 hour. The actin filaments and cell nuclei were then stained with phalloidin-iFluor 488 (Abcam) and DAPI (Sigma-Aldrich) respectively according to manufacturer's instructions. The imaging was performed with THUNDER fluorescence imagining system (Leica).

### Example 13 - Cytocompatibility test

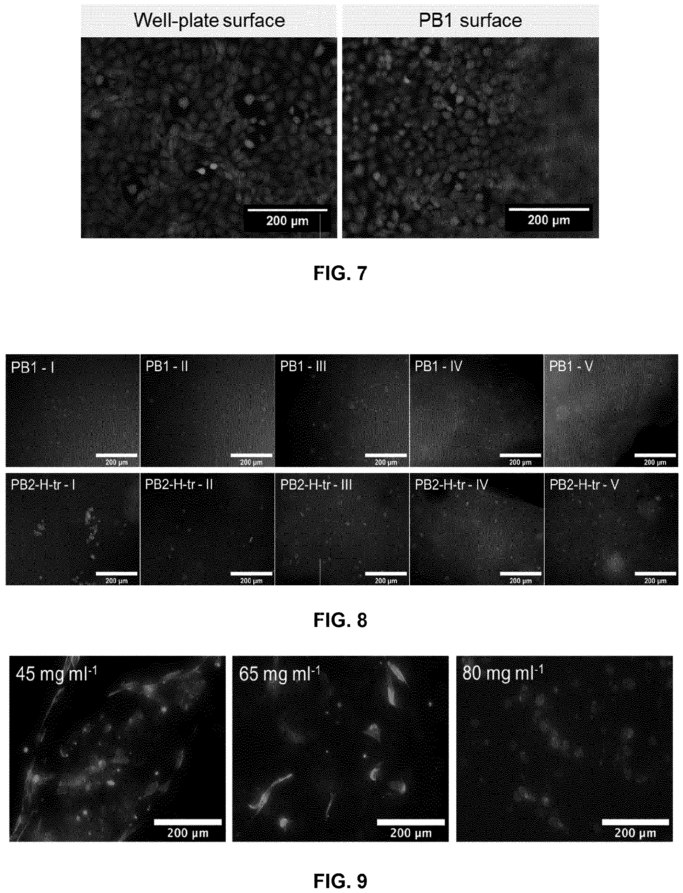

For cell types and cultivation see Example 11. Cytocompatibility tests were evaluated with three different set ups. First, cytotoxicity was evaluated by culturing cells together with protein hydrogel material. Second, the cells were seeded on top of protein gels and in the third version the cells were incapsulated in the 3D hydrogel. All gels were prepared by the method described in example 7. The cells (2 × 10⁶ cells mL⁻¹) were encapsulated in hydrogels prepared at concentrations described in **Table 5**. For cells cultured together with hydrogel no significant difference was observed compared to control (2D culture of cells without gel). Cells seeded on the top has a similar engagement with the material (cell morphology indicative of cell spreading) to the morphology of cells cultured on the 2D well-plate surface (control group) (**Figure 7**).

During the 7 days of incubation of NIH/3T3 cell in the hydrogels, we observed that cells encapsulated in high protein concentration scaffolds had more elongated shapes, whereas in low concentration gels the cells were more rounded (**Figure 8**).

**Table 5. Protein PB1 and PB2-H-tr solution concentration c before dialysis and storage modulus G' after dialysis. The gels were used for cell encapsulation experiments.**

| **PB1** | **C, mg mL⁻¹** | **G', kPa** | **PB2-H-tr** | **C, mg mL⁻¹** | **G', kPa** |
|---|---|---|---|---|---|
| **I** | 30 | 0.2 - 0.3 | **I** | 34 | <0.1 |
| **II** | 54 | 1 | **II** | 26 | 0.8 |
| **III** | 111 | 5 | **III** | 79 | - |
| **IV** | 122 | - | **IV** | 100 | 18 |
| **V** | 141 | 10 | **V** | 134 | 34 |

The studies demonstrated that most of the cells were alive, more than 80 % based on Live/Dead staining (Viability/Cytotoxicity kit, ThermoFisher) after 3 days.

Protein gel concentration screening was performed for proteins of the A-B-A-B-A and A-B-B architecture to identify suitable conditions that maintained cell viability and allow cell. hMSCs were investigated and in the experiments viable cells after 5 days were observed. Cell spreading was observed in lower concentration gels (45 and 65 mg mL⁻¹) (storage modulus G' < 2 kPa)(**Figure 9**). Cytocompatibility tests performed *inter alia* with PB0, PB1, PB2-H-tr and MMP2-PB2, are summarised in **Table 6**.

**Table 6. Summary of the cytocompatibility tests and their outcome while using the three proteins PB0, PB1 and PB2-H-tr.**

| **Protein** | **Test*, cell type** | **Protein concentration, mg mL⁻¹** | **Duration, days** | **Outcome** |
|---|---|---|---|---|
| PB0 | (3) | 30 | 14 | cells viable |
| PB1 | (1), NIH/3T3 | 70 | 2 | cells viable |
| | (2), N I H/3T3 | 70 | 2 | cells viable |
| | (3), N I H/3T3 | 10-100 | 2-3 | cells viable |
| | (3), C2C12 | 80-90 | 14 | cells viable |
| | (3), hMSCs | 20-100 | 14-21 | cells viable |
| PB2-H-tr | (2), N I H/3T3 | 50 | 1-3 | cells viable |
| | (3), N I H/3T3 | 10-100 | 14-21 | cells viable |
| | (3), C2C12 | 50-80 | 14 | cells viable |
| | (3), hMSCs | 20-100 | 21 | cells viable |
| MMP2-PB2 | (3), hMSCs | 60 | 20 | cells viable |

| | | | | |
|---|---|---|---|---|
| *Protein cytocompatibility tests: (1) Seeding the cells in the same well as protein hydrogel 2D; (2) Seeding the cells on top of the hydrogel 2D; (3) Encapsulating the cells in the hydrogel 3D. | | | | |

### Example 14 - Cell viability in printed samples

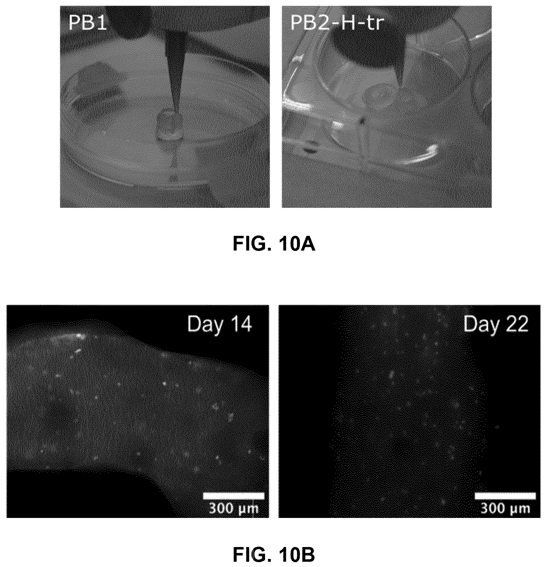

3D structures were printed using 1 × 10⁶ cells mL⁻¹ hMSCs and 87.5 mg mL⁻¹ initial concentration PB1 protein hydrogel and 101 mg mL⁻¹ initial concentration PB2-H-tr protein hydrogel. Similar printing parameters to the ones described in Example 10 were used, and a bigger printing nozzle was used (conical, 22GA, d = 410 mm). The addition of cells did not compromise gel stability and it was possible to print 3D structures with both bioinks (**Figure 10A**). As expected, cells were viable in the printed samples (based on live/dead staining; **Figure 10B**).

## Claims

1. A hydrogel-forming protein comprising:
at least three self-assembling aggregation domains and
at least two hydrophilic non-self-assembling linker domains,
wherein the self-assembling aggregation domains are covalently interconnected by the hydrophilic non-self-assembling linker domain, either directly or via a peptide linker.

2. The hydrogel-forming peptide of any one of the preceding claims having a length of 100 to 300 amino acids, preferably of 150 to 260 amino acids, more preferably 170 to 260 amino acids.

3. The hydrogel-forming protein any one of the preceding claims, wherein at least one, preferably all, of the self-assembling aggregation domains are amphiphilic beta-sheet forming peptide domains.

4. The hydrogel-forming protein of any one of the preceding claims comprising 3 to 5, preferably 3 or 4, most preferably 3 self-assembling aggregation domains.

5. The hydrogel-forming protein of any one of the preceding claims, wherein each of the aggregation domains has a length from about 16 to about 60, preferably from about 24 to about 50, more preferably from about 24 to about 40, again more preferably about 40 amino acids.

6. The hydrogel-forming protein of any one of the preceding claims, wherein said self-assembling aggregation domains each comprises at least two repeat units, preferably 2 to 8 repeat units, more preferably 3 to 6 repeat units, again more preferably 3 to 5 repeat units, most preferably 3 or 5 repeat units, again more preferably 5 repeat units.

7. The hydrogel-forming protein of any one of the preceding claims, wherein said repeat unit has an amino acid sequence selected of the group consisting of SEQ ID NOs: 1-20, more preferably of SEQ ID NOs: 1-4, 7, 10, most preferably, the repeat unit has an amino acid sequence of SEQ ID NO: 1.

8. The hydrogel-forming protein of any one of the preceding claims comprising 2 to 4, preferably 2 or 3, most preferably 2 hydrophilic non-self-assembling linker domains.

9. The hydrogel-forming protein of any one of the preceding claims, wherein the hydrophilic linker domains each has a length of more than 30 amino acids, and wherein preferably the hydrophilic linker domains have a random-coil or unstructured conformation.

10. The hydrogel-forming protein of any one of the preceding claims, wherein the hydrophilic linker domain comprises at least one linker repeat unit comprising the sequence GXGSGSG (SEQ ID NO: 70) or GXGSGSGRGDS (SEQ ID NO: 71), wherein X represents a charged or polar amino acid selected from the group consisting of lysine, arginine, histidine, aspartic acid, glutamic acid, serine, homoserine, threonine, glutamine, and asparagine; wherein preferably X is lysine or serine.

11. The hydrogel-forming peptide of any one of the preceding claims, wherein the hydrophilic linker domain comprises from 2 to 7 linker repeat units, preferably the linker repeat unit is selected from the group consisting of SEQ ID NO: 21-27 and 72.

12. The hydrogel-forming protein of any one of the preceding claims, wherein the hydrophilic linker domain comprises at least one cell-binding domain, preferably 2 to 7 cell-binding domain, wherein preferably the cell-binding domain is selected from the group consisting of RGD, RGDS (SEQ ID NO: 29), PHSRN (SEQ ID NO: 31) and PHSRNS (SEQ ID NO: 32).

13. The hydrogel-forming protein of any one of the preceding claims having the formula of A-B-A-B-A, wherein A is the self-assembling aggregation domain, covalently linked to B, either directly or via a peptide linker, wherein B is the hydrophilic non-self-assembling linker domain.

14. The hydrogel-forming protein of any one of the preceding claims, having a sequence selected from the group consisting of SEQ ID NO: 58-65.

15. A bioink comprising of the hydrogel-forming peptide of any one of the preceding claims for three-dimensional (3D) bio-fabrication.
